# EUROPÄISCHE PATENTSCHRIFT

(11) **EP 2 184 028 B1**
(45) Veröffentlichungstag und Bekanntmachung des Hinweises auf die Patenterteilung: **05.06.2013**
(21) Anmeldenummer: 08019312.1
(22) Anmeldetag: 05.11.2008
(51) Int. Cl.: G06K 19/077, A61C 1/00, A61C 1/08, A61C 17/22, A61B 1/00, A61B 18/14, G06K 7/08

(54) **Medizinische, insbesondere dentale, Behandlungsvorrichtung**
Medical, in particular dental, treatment device
Instrument de traitement médical, en particulier dentaire

(43) Veröffentlichungstag der Anmeldung: 12.05.2010
(73) Patentinhaber: W & H Dentalwerk Bürmoos GmbH, 5111 Bürmoos (AT)
(72) Erfinder: Pruckner, Christian, 1190 Wien (AT); Brandstätter, Andreas, 5120 St. Pantaleon (AT); Wendtner, Wolfgang, 5112 Lamprechtshausen (AT)
(74) Vertreter: Benda, Ralf

(56) Entgegenhaltungen:
- WO-A-2008/021507
- DE-A1- 3 706 934
- DE-C- 3 708 801
- GB-A- 2 382 876
- US-A1- 2007 234 493

## Beschreibung

Die vorliegende Erfindung betrifft eine medizinische, insbesondere dentale, Behandlungsvorrichtung mit einer Werkzeugerkennungsvorrichtung zur Erkennung ob ein Werkzeug an die Behandlungsvorrichtung angeschlossen ist oder welches von mehreren unterschiedlichen Werkzeugen an die Behandlungsvorrichtung angeschlossen ist und ein Verfahren zur Erkennung ob ein Werkzeug an eine medizinische, insbesondere dentale, Behandlungsvorrichtung angeschlossen ist oder welches der unterschiedlichen Werkzeuge an die Behandlungsvorrichtung angeschlossen ist.

Eine derartige Behandlungsvorrichtung ist zum Beispiel aus der Patentschrift DE 37 08 801 C2 bekannt. Sie umfasst unter anderem ein Handstück und eine Werkzeugerkennungsvorrichtung zur Erkennung unterschiedlicher, mit der Behandlungsvorrichtung verbindbarer Werkzeuge. Die Werkzeuge weisen jeweils einen Ferritkern und unterschiedliche Spulen auf. In der Behandlungsvorrichtung ist eine Messspule vorgesehen, die von einer Wechselspannungsquelle mit gewobbelter Wechselspannung versorgt wird und die mit den unterschiedlichen Werkzeugen induktiv koppelbar ist. Aufgrund der für jedes Werkzeug typischen Eingangsimpedanz weist das Wechselstromsignal nach der Spule einen ebenfalls typischen frequenzabhängigen Verlauf auf. Eine Mess- und Auswerteinrichtung misst nach dem Verbinden eines Werkzeugs mit dem Handstück unter Berücksichtigung der jeweiligen Wechselspannungsfrequenz die in Abhängigkeit von dem jeweils am Handstück vorgesehenen Werkzeug unterschiedliche elektrische Impedanz des Handstückes und erkennt auf Basis dieser Impedanzmessung das Werkzeug.

Der Nachteil dieser Werkzeugerkennungsvorrichtung ist ihr mess- und auswertungstechnisch komplizierter Aufbau, insbesondere die mehrfache Messung bei unterschiedlichen Frequenzen und die Notwendigkeit jedes Werkzeug mit einer unterschiedlichen Spule zu versehen. Der vorliegenden Erfindung liegt daher die Aufgabe zugrunde eine Behandlungsvorrichtung mit einer mess- und auswertungstechnisch einfacher aufgebauten Werkzeugerkennungsvorrichtung zu schaffen.

Diese Aufgabe wird gemäß einer medizinischen, insbesondere dentalen Behandlungsvorrichtung gemäß Anspruch 1 gelöst. Bevorzugt ist die Spule mit ihrem eingangsseitigen Ende mit einer Wechselspannungsquelle und mit ihrem ausgangsseitigen Ende mit einer Auswerteeinheit zur Auswertung des ausgangsseitig der Spule anliegenden Wechselspannungssignals verbindbar oder verbunden.

Die Werkzeugerkennung erfolgt ausschließlich auf Basis des durch das Werkzeug veränderten Wechselspannungssignals, wodurch der Aufbau der Mess- und Auswerteeinheit merklich vereinfacht ist. Die Veränderung, d.h. die Vergrößung oder die Verkleinerung, des Wechselspannungssignals wird durch die weichmagnetischen Eigenschaften der Werkzeuge verursacht, welche die Induktivität der Spule beeinflussen. Die Erkennung, ob ein Werkzeug an die Behandlungsvorrichtung angeschlossen ist, basiert auf dem gleichen Messprinzip, wobei die Auswerteeinheit in diesem Fall nur zwischen einem ersten Wechselspannungssignal, das im Wesentlichen gleich dem eingangsseitig der Spule anliegenden Wechselspannungssignals ist und das vorliegt, wenn kein Werkzeug an die Behandlungsvorrichtung angeschlossen ist, und einem zweiten Wechselspannungssignal, das von dem eingangsseitig der Spule anliegenden Wechselspannungssignals abweicht und das vorliegt, wenn ein Werkzeug an die Behandlungsvorrichtung angeschlossen ist, unterscheidet.

Gemäß einem Ausführungsbeispiel weisen die den jeweiligen Werkzeugen zuordenbaren Wechselspannungssignale unterschiedliche Amplituden mit unterschiedlichen Amplitudenextremwerten auf, wobei die Auswerteeinheit zur Unterscheidung der Werkzeuge auf der Basis zumindest eines Amplitudenextremwerts ausgebildet ist. Die Verwendung der Amplitudenextremwerte, d.h. der Spannungsmaxima und / oder der Spannungsminima garantiert eine besonders zuverlässige und exakte Unterscheidung der verschiedenen Werkzeuge.

Gemäß einem bevorzugten Ausführungsbeispiel weist die Auswerteeinheit eine Wandlereinheit auf, die insbesondere zumindest einen Gleichrichter und einen Glättungskondensator umfasst, welche die den jeweiligen Werkzeugen zuordenbaren Wechselspannungssignale in Gleichspannungssignale wandelt. Besonders bevorzugt ist der Spannungswert der Gleichspannungssignale in etwa gleich dem Spannungswert zumindest eines Amplitudenextremwerts der jeweiligen Wechselspannungssignale.

Die Auswertung der Wechselspannungssignale oder der daraus abgeleiteten Gleichspannungssignale auf der Basis zumindest eines Amplitudenextremwerts / Spannungsextremwerts erfolgt bevorzugt als Spitzen-Spitzen-Erkennung, bei der die Auswerteeinheit die Spannungsdifferenz zwischen den maximalen Spannungswerten und den minimalen Spannungswerten ermittelt. Alternativ bestimmt die Auswerteeinheit die Spannungsdifferenz zwischen den Amplitudenextremwerten / Spannungsextremwerten und einem feststehenden Wert, insbesondere dem Nullpunkt. Dazu werden entweder nur eine Halbwelle der sinusförmigen Wechselspannungssignale oder beide Halbwellen verwendet. Werden die Wechselspannungssignale vor der Auswertung gleichgerichtet, so erfolgt dies durch Einweggleichrichtung bei Verwendung nur einer Halbwelle oder Vollweggleichrichtung bei Verwendung beider Halbwellen, wodurch ein stabileres Signal entsteht. Unabhängig von der verwendeten Auswertungsmethode weist jedes Werkzeug einen spezifischen, dem jeweiligen Werkzeug zuordenbaren Spannungsdifferenzwert oder einen dem jeweiligen Werkzeug zuordenbaren Betrag des Spannungsdifferenzwerts auf.

Um für jedes Werkzeug, insbesondere für Werkzeuge die zu einem Werkzeugsatz zusammengefasst sind und die gegebenenfalls sequentiell oder alternierend während einer medizinischen Behandlung verwendet werden, ein unterschiedliches, dem jeweiligen Werkzeug eindeutig zuordenbares Wechselspannungssignal zu erhalten, weisen die Werkzeuge unterschiedliche Geometrien auf. Insbesondere unterscheiden sich die Werkzeuge in ihren Schaftlängen oder in ihren Durchmessern, besonders bevorzugt in den Durchmessern der Werkzeugschäfte. Alternativ oder zusätzlich unterscheiden sich die Werkzeuge in den Materialien, aus denen sie bestehen oder hergestellt sind. Bevorzugt bestehen die Werkzeuge aus unterschiedlichen weichmagnetischen Materialien, zum Beispiel aus unterschiedlichen Stählen. Es ist jedoch auch denkbar, dass zur Unterscheidung zumindest eines Werkzeugs dieses aus einem nicht-weichmagnetischen Material besteht, zum Beispiel aus Kunststoff. Bei einem derartigen Werkzeug handelt es sich zum Beispiel um eine Stopfsonde für Zahnfüllmaterial.

Aufgrund der unterschiedlichen Geometrien oder Materialien der Werkzeuge ist es in vorteilhafter Weise nicht notwendig, die Werkzeuge zur Unterscheidung mit weiteren Bauteilen wie Spulen oder Markierungen zu versehen.

Gemäß einem bevorzugten Ausführungsbeispiel umfasst ein Werkzeugsatz fünf unterschiedliche Werkzeuge, insbesondere Werkzeuge zur Entfernung von Zahnstein oder Zahnbelag. Jedes Werkzeug weist einen Arbeitsabschnitt zur Einwirkung auf eine Behandlungsstelle und einen daran anschließenden Schaft zur Verbindung oder Kupplung des Werkzeugs mit der Behandlungsvorrichtung auf. Die Werkzeugschäfte weisen Durchmesser zwischen in etwa 3,5 - 5,5 mm auf, insbesondere zwischen 4,0 - 5,0 mm, wobei jeder Werkzeugschaft einen eigenen, spezifischen Durchmesser aufweist. Um trotz des nicht-linearen Verlaufs der Induktivität eine eindeutige Unterscheidung der Werkzeuge zu gewährleisten, sind gemäß einem besonders bevorzugt Ausführungsbeispiel die Differenzbeträge zwischen den Durchmessern aufeinander folgender Werkzeugschäfte unterschiedlich, zum Beispiel betragen die Durchmesser in etwa 4,10 mm, 4,20 mm, 4,40 mm, 4,65 mm und 4,90 mm und die Differenzbeträge zwischen den Durchmessern der aufeinander folgenden Werkzeugschäfte somit etwa 0,10 mm, 0,20 mm oder 0,25 mm.

Die Werkzeugserkennungsvorrichtung ist zur Unterscheidung oder Erkennung unterschiedlicher Arten von Werkzeugen geeignet, zum Beispiel von Werkzeugen die für eine rotierende, eine schwingende, eine oszillierende oder eine hubartige Arbeitsbewegung bestimmt sind. Dem gemäß umfasst die Antriebseinheit zum Antrieb der Werkzeuge Antriebe oder Motore, die eine rotierende oder schwingende Bewegung erzeugen, zum Beispiel Elektromotore, mit Fluid, insbesondere Druckgas, betreibbare Antriebe oder piezoelektrische oder magnetostriktive Schwingungserreger. Die Antriebseinheit umfasst des Weiteren Wellen, Schwingachsen, Getriebe, Zahnräder, Kupplungen etc. zur Übertragung und / oder Umwandlung der von den Antrieben oder Motoren erzeugten Antriebsbewegung.

Die Behandlungsvorrichtung umfasst neben der Antriebseinheit bevorzugt ein mit einer Hand fassbares Handgriffelement, zum Beispiel ein medizinisches, insbesondere dentales, Hand- oder Winkelstück oder ein pistolenförmiges Griffstück, eine Energiequelle oder einen Anschluss an eine Energiequelle zur Energieversorgung der Behandlungsvorrichtung, einen oder mehrere Medienquellen oder Anschlüsse an eine oder mehrere Medienquellen, insbesondere an eine Wasserversorgung und an eine Druckluftquelle, und Versorgungsleitungen zwischen dem Handgriffelement und der Energiequelle bzw. der zumindest einen Medienquelle. Die Werkzeugserkennungsvorrichtung ist entweder zur Gänze im Handgriffelement angeordnet oder es sind zumindest Teile davon, insbesondere die Auswerteeinheit, die bevorzugt einen Mikrocontroller aufweist, oder die Energiequelle, außerhalb des Handgriffelements angeordnet, zum Beispiel in einem eigenständigen Steuergerät, das über einen Versorgungsschlauch mit dem Handgriffelement verbunden ist.

Die Erkennung und Unterscheidung der unterschiedlichen Werkzeuge dient bevorzugt dazu, der Behandlungsvorrichtung, insbesondere dem Handgriffelement, der Antriebseinheit oder dem Werkzeug, auf das Werkzeug abgestimmte Betriebsmittel oder Betriebsmittelmengen zukommen zu lassen. So weisen zum Beispiel schwingend betriebene Werkzeuge unterschiedliche Resonanzfrequenzen auf, wobei die Schwingungen erzeugende Antriebseinheit abhängig von dem an die Behandlungsvorrichtung angeschlossenen Werkzeug mit unterschiedlichen Versorgungsspannungen versorgt wird, so dass das angeschlossene Werkzeug möglichst exakt mit seiner Resonanzfrequenz betrieben werden kann. Gemäß einem bevorzugten Ausführungsbeispiel ist die Behandlungsvorrichtung als Zahnsteinentfernungs- oder Scalerbehandlungsvorrichtung ausgebildet, deren Antriebseinheit einen, insbesondere piezoelektrischen, Schwingungserreger umfasst, der in Abhängigkeit des mit der Behandlungsvorrichtung verbundenen und von der Werkzeugerkennungsvorrichtung erkannten Werkzeugs mit auf das Werkzeug abgestimmter Antriebsenergie, Antriebsleistung oder Antriebsspannung versorgbar ist.

Alternativ oder zusätzlich benötigen unterschiedliche Werkzeuge unterschiedliche Kühlmittel oder Kühlmittelmengen, so dass die Versorgungseinheit auf das Werkzeug abgestimmte Kühlmittel, zum Beispiel eine Kühlflüssigkeit oder ein Druckgas, oder Kühlmittelmengen abgibt.

Damit die Versorgungseinheit die auf das Werkzeug abgestimmten Betriebsmittel oder Betriebsmittelmengen abgeben kann, ist die Werkzeugerkennungsvorrichtung, insbesondere die Auswerteeinheit, nach der Erkennung des an die Behandlungsvorrichtung angeschlossenen Werkzeugs zur Abgabe eines spezifischen Steuersignals für jedes Werkzeug an eine Versorgungseinheit ausgebildet. Dazu sind in der Auswerteeinheit ein Speicher, in dem für jedes Werkzeug ein charakteristischer Wert hinterlegt ist, insbesondere ein charakteristischer Spannungswert, und einen Komparator vorgesehen, der zur Erkennung des mit der Behandlungsvorrichtung verbundenen Werkzeugs den hinterlegten Wert mit dem für jedes Werkzeug spezifischen Wechselspannungssignal oder einem davon abgeleiteten Signal vergleicht. In der Versorgungseinheit ist ein weiterer Speicher vorgesehen, auf dem für jedes Werkzeug Daten über die benötigten Betriebsmittel oder Betriebsmittelmengen hinterlegt sind. Auf Basis dieser Daten steuert die Versorgungseinheit die Abgabe der Betriebsmittel oder Betriebsmittelmengen. Selbstverständlich können die beiden Speicher als ein gemeinsamer Speicher ausgebildet sein, der Teil der Behandlungsvorrichtung ist, insbesondere des Steuergeräts.

Gemäß einem weiteren Ausführungsbeispiel weist die Behandlungsvorrichtung eine Beleuchtungsvorrichtung mit zumindest einem optischen Halbleiterelement auf, insbesondere mit zumindest einer Leuchtdiode (LED), wobei zur Versorgung der Beleuchtungsvorrichtung und der Spule der Werkzeugerkennungsvorrichtung mit elektrischer Energie eine gemeinsame elektrische, insbesondere zweiadrige, Leitung vorgesehen ist und wobei die Spule und die Beleuchtungsvorrichtung elektrisch parallel geschaltet sind. Durch diesen äußerst Platz sparenden Aufbau ist es möglich, die Werkzeugerkennungsvorrichtung und die Beleuchtungsvorrichtung in unmittelbarer Nähe zur Werkzeugaufnahme oder Werkzeugkupplung der Behandlungsvorrichtung anzuordnen. Bevorzugt ist die Beleuchtungsvorrichtung, die zum Beispiel mehrere LEDs umfasst, rund um die Werkzeugkupplung angeordnet.

Bevorzugt ist die gemeinsame elektrische Leitung mit einer Energiequelle verbindbar oder verbunden, die zur Versorgung der Beleuchtungsvorrichtung Gleichspannung und zur Versorgung der Spule der Werkzeugerkennungsvorrichtung Wechselspannung bereitstellt. Damit die gemeinsame elektrische Leitung die Beleuchtungsvorrichtung mit Gleichspannung und die Spule mit Wechselspannung versorgen kann, wird die Wechselspannung der Gleichspannung aufmoduliert oder überlagern die beiden Spannungen einander. Damit wird die Anordnung der Spule und der Beleuchtungsvorrichtung im räumlich sehr beengten Umfeld rund um die Werkzeugaufnahme oder Werkzeugkupplung weiter vereinfacht, da die Verwendung zusätzlicher elektrischer oder elektronischer Bauteile in diesem Bereich, zum Beispiel eines AC-DC-Wandlers, entfallen kann.

Gemäß einem bevorzugten Ausführungsbeispiel weist die Energiequelle eine Gleichspannungsquelle, einen Rechteck- oder Sinusoszillator, einen Kondensator für die Versorgung der Spule mit Wechselspannung und einen parallel zum Kondensator geschalteten Widerstand für die Versorgung des zumindest einen optischen Halbleiterelements mit Gleichspannung auf. Die gemeinsame elektrische Leitung verbindet diese Energiequelle mit der Spule und der Beleuchtungsvorrichtung. Selbstverständlich kann die Energiequelle jedoch auch anders aufgebaut sein und zum Beispiel eine Gleichspannungsquelle und eine davon unabhängige Wechselspannungsquelle aufweisen. Zumindest eine der beiden Spannungsquellen kann eine Batterie oder einen Akkumulator umfassen.

Gemäß einem besonders bevorzugten Ausführungsbeispiel umfasst die Behandlungsvorrichtung ein Schaltelement zum Schalten der Energieversorgung für die Spule und die Beleuchtungsvorrichtung zwischen einem ersten Betriebszustand, bei dem die Werkzeugerkennung erfolgt, und einem zweiten Betriebszustand, wobei während des ersten Betriebszustands die Gleichspannung einen niedrigen Spannungswert und die Wechselspannung einen hohen Spannungswert aufweisen und wobei während des zweiten Betriebszustands die Gleichspannung einen hohen Spannungswert und die Wechselspannung einen niedrigen Spannungswert aufweisen. Während des zweiten Betriebszustands wird zum Beispiel das Werkzeug angetrieben. Das Schalten zwischen diesen beiden Betriebszuständen bzw. den unterschiedlichen Spannungswerten garantiert eine optimale Werkzeugerkennung und einen optimalen Betrieb der Beleuchtungsvorrichtung. Insbesondere schützt die Verringerung der Gleichspannung die Leuchtdioden während der Werkzeugerkennung vor einer zu hohen Spannungsversorgung und die Werkzeugerkennung vor einer Beeinflussung oder Verfälschung der Messsignale.

Gemäß einem Ausführungsbeispiel beträgt der niedrige Spannungswert der Gleichspannung pro optischem Halbleiterelement zwischen etwa 0,9 V - 1,5 V, bevorzugt etwa 1,1 - 1,2 V, der hohe Spannungswert der Gleichspannung pro optischem Halbleiterelement zwischen etwa 2,5 V - 3,3 V, bevorzugt etwa 3,0 - 3,2 V, der niedrige Spannungswert der Wechselspannung zwischen etwa 0,0 V - 0,5 V und dass der hohe Spannungswert der Wechselspannung zwischen etwa 10 V - 18 V, bevorzugt etwa 15 V.

Bevorzugt wird vor Beginn der Werkzeugerkennung oder nach Inbetriebnahme der Behandlungsvorrichtung die Gleichspannung für die LEDs auf den niedrigen Spannungswert und anschließend die Wechselspannung für die Spule der Werkzeugerkennungsvorrichtung auf den hohen Spannungswert geschaltet. Nach Abschluss der Werkzeugerkennung wird zuerst die Wechselspannung auf den niedrigen Spannungswert geschaltet und anschließend die Gleichspannung auf den hohen Spannungswert geschaltet. Das Schaltelement ist bevorzugt als elektronischer Schaltkreis ausgebildet, der insbesondere Teil des Mikrocomputers im Steuergerät ist.

Ein Verfahren zur Erkennung, ob ein Werkzeug an eine im Vorherstehenden beschriebene medizinische, insbesondere dentale, Behandlungsvorrichtung angeschlossen ist oder welches der unterschiedlichen Werkzeuge an die Behandlungsvorrichtung angeschlossen ist, umfasst den Schritt, dass die Spule mit einem der unterschiedlichen Werkzeugen induktiv gekoppelt wird, wodurch ein für jedes Werkzeug spezifisches, durch die Auswerteeinheit dem jeweiligen Werkzeug zuordenbares Wechselspannungssignal generiert wird. Bevorzugt weist die Behandlungsvorrichtung eine Beleuchtungsvorrichtung mit zumindest einem optischen Halbleiterelement auf, wobei gemäß einem weiteren Verfahrensschritt die Beleuchtungsvorrichtung und die Spule der Werkzeugerkennungsvorrichtung, die parallel zueinander angeordnet sind, über eine gemeinsame elektrische Leitung mit elektrischer Energie versorgt werden. Insbesondere wird die Energieversorgung für die Spule und die Beleuchtungsvorrichtung zwischen einem ersten Betriebszustand, bei dem die Werkzeugerkennung erfolgt, und einem zweiten Betriebszustand geschaltet, wobei während des ersten Betriebszustands die Gleichspannung einen niedrigen Spannungswert und die Wechselspannung einen hohen Spannungswert aufweisen und wobei während des zweiten Betriebszustands die Gleichspannung einen hohen Spannungswert und die Wechselspannung einen niedrigen Spannungswert aufweisen.

Die Erfindung wird nachfolgend anhand bevorzugter Ausführungsbeispiele und Bezug nehmend auf die beigefügten Zeichnungen erläutert:
Figur 1 zeigt ein Ausführungsbeispiel einer medizinischen, insbesondere dentalen, Behandlungsvorrichtung mit einer Werkzeugerkennungsvorrichtung und einem Handgriffelement zur Zahnsteinentfernung.
Figur 2 zeigt eine Schnittdarstellung des werkzeugseitigen Endes des Handgriffelements zur Zahnsteinentfernung der Figur 1.
Figur 3 zeigt in einer schematischen Darstellung ein Ausführungsbeispiel eines Schaltbilds der Werkzeugerkennungsvorrichtung.
Figur 4 zeigt ein Ausführungsbeispiel einer Auswerteeinheit der Behandlungsvorrichtung zur Auswertung der Wechselspannungssignale.

Die Figuren 5A und 5B zeigen zwei unterschiedliche, an eine medizinische, insbesondere dentale, Behandlungsvorrichtung anschließbare Werkzeuge mit unterschiedlichen Schaftdurchmessern zur Unterscheidung durch eine Werkzeugerkennungsvorrichtung.

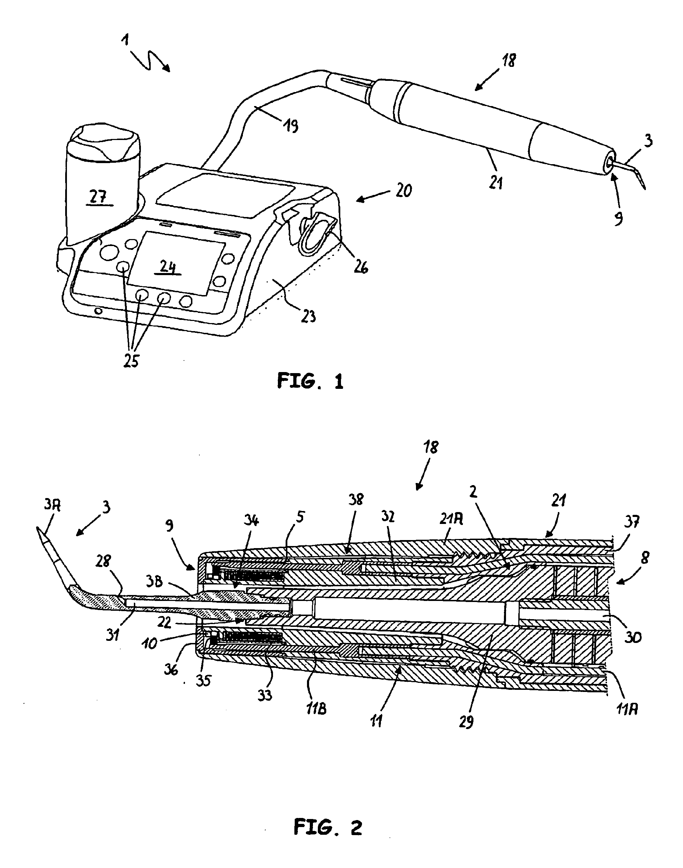

Die in Figur 1 dargestellte medizinische, insbesondere dentale, Behandlungsvorrichtung 1 ist als Zahnsteinentfernungs- oder Scalerbehandlungsvorrichtung ausgebildet. Sie besteht aus einem Handgriffelement oder Handstück 18, einem Steuergerät 20 und einem Versorgungs- oder Verbindungsschlauch 19, der das Steuergerät 20 und das Handstück 18 verbindet.

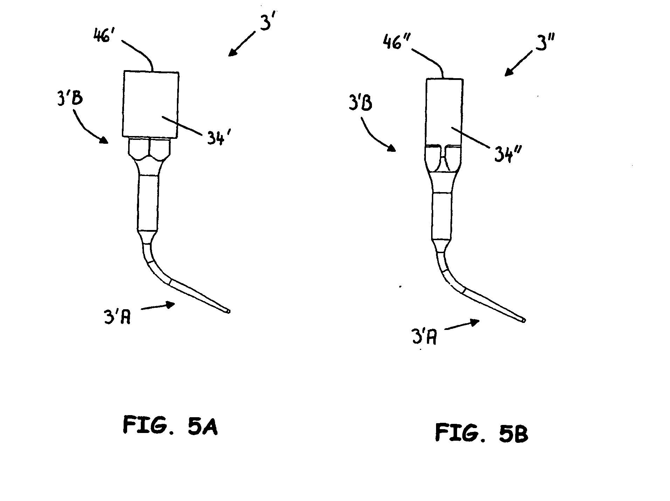

Das gerade, längliche Handstück 18 weist eine zylindrische, hohle Außenhülse 21 auf, in der, wie im Weiteren noch im Detail beschrieben, unter anderem die Antriebseinheit 2 für ein mit dem Handstück 18 verbindbares Werkzeug 3, 3', 3" (siehe Figuren 5A, 5B), zumindest Teile der Werkzeugerkennungsvorrichtung 4, eine Werkzeugaufnahme 22 zur lösbaren Aufnahme unterschiedlicher Werkzeuge 3, 3', 3", und eine die Werkzeugaufnahme 22 umgebende Beleuchtungsvorrichtung 9 angeordnet sind. Das Steuergerät 20 weist ein Gehäuse 23 mit einer Anzeige 24 für feststehende oder einstellbare Betriebsparameter oder für das von der Werkzeugerkennungsvorrichtung 4 detektierte Werkzeug 3, 3', 3", ein oder mehrere Stellelemente 25, zum Beispiel Drucktaster, zur Auswahl oder Änderung von Betriebsparametern, eine Handstückablage 26 und eine Flüssigkeitsquelle 27 mit einer Kühl- oder Spülflüssigkeit auf.

Der Versorgungs- oder Verbindungsschlauch 19 enthält mehrere Medien- oder Betriebsmittelleitungen, insbesondere elektrische Leitungen, die die Antriebseinheit 2 und die Werkzeugerkennungsvorrichtung 4 mit entsprechenden Energiequellen, zum Beispiel der Energiequelle 12, verbindet. Gemäß einem bevorzugten Ausführungsbeispiel weist die Behandlungsvorrichtung zwei vollständig voneinander getrennte, elektrische Versorgungskreise auf, wobei ein elektrischer Versorgungskreis die Antriebseinheit 2 mit elektrischer Energie versorgt und der andere elektrische Versorgungskreis die Werkzeugerkennungsvorrichtung 4 mit elektrischer Energie versorgt. Eine Medienleitung verbindet die Flüssigkeitsquelle 27 mit der Werkzeugaufnahme 22 und einem darin aufgenommenen Werkzeug 3, 3', 3", so dass über eine Flüssigkeitsabgabeöffnung 28 des Werkzeugs 3, 3', 3" Flüssigkeit auf die Behandlungsstelle und / oder das Werkzeug 3, 3', 3", insbesondere dessen Arbeitsabschnitt 3A, 3'A, 3"A abgebbar ist.

Wie aus der Figur 2 zu erkennen ist, umfasst die Antriebseinheit einen piezoelektrischen Schwingungserreger 8 mit mehreren Piezoelementen und eine mit dem Schwingungserreger 8 verbundene Sonotrode 29, die als hohler Schwingschaft ausgebildet ist. An einem Ende der Sonotrode 29 ist die Werkzeugaufnahme 22 zur lösbaren Aufnahme unterschiedlicher Werkzeuge, zum Beispiel der Werkzeuge 3, 3', 3", vorgesehen. Die Werkzeugaufnahme 22 umfasst zum Beispiel ein Innengewinde, das mit einem Außengewinde des Werkzeugs 3 das insbesondere an dessen Werkzeugschaft 3B vorgesehen ist, verbindbar ist. Die Werkzeugaufnahme 22 kann auch eine konische Reibfläche aufweisen, die mit einer zweiten konischen Reibfläche des Werkzeugs 3 die insbesondere an dessen Werkzeugschaft 3B vorgesehen ist, einen Reibschluss bildet. Die in den Figuren 5A und 5B dargestellten alternativen Werkzeuge 3', 3" weisen am Werkzeugschaft 3'B, 3"B ein Innengewinde auf, das mit einem entsprechenden Außengewinde einer Werkzeugaufnahme verbindbar ist.

Die mit der Flüssigkeitsquelle 27 verbundene Medienleitung 30 mündet in die hohle Sonotrode 29, von der die Kühlflüssigkeit in einen Kanal 31 des Werkzeugs 3 übertritt, um über die Flüssigkeitsabgabeöffnung 28 auszutreten.

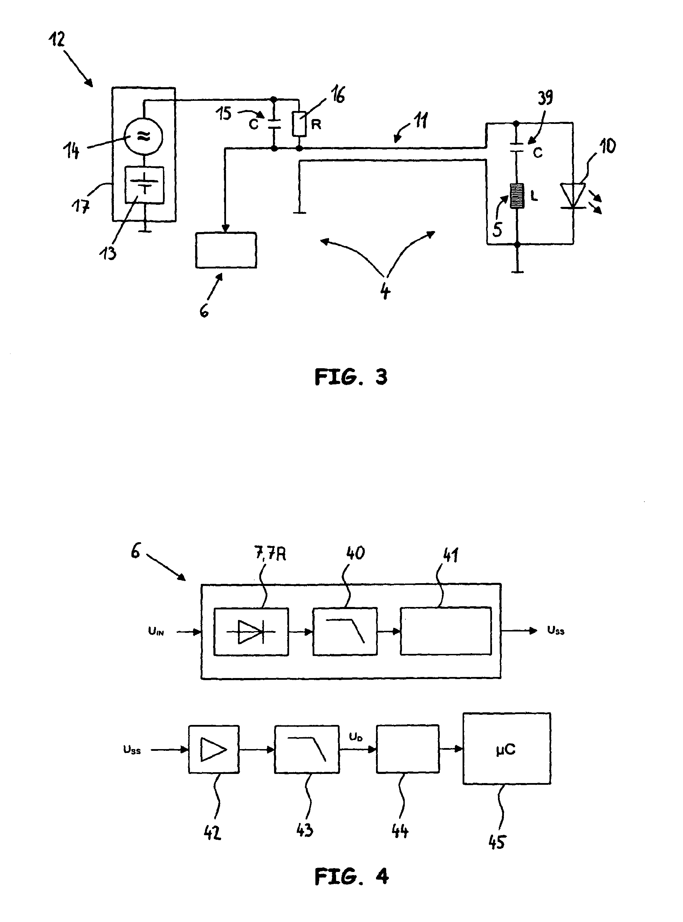

Das werkzeugseitige Ende der Sonotrode 29, die Werkzeugaufnahme 22 und ein darin aufgenommener Werkzeugschaft 3B, 3'B, 3"B sind von der Beleuchtungsvorrichtung 9 und einer Spule 5 umgeben, die Teil der Werkzeugerkennungsvorrichtung 4 (siehe Figur 3) ist. Die zylindrische Spule 5 ist auf einer Trägerhülse 32 aus Kunststoff gelagert. Ein magnetisches Rückschlusselement 33, das bevorzugt aus Metallblech besteht, umgibt die Spule an ihrer Außenseite und zum Teil auch an ihrer dem Werkzeugschaft 3B, 3'B, 3"B und der Sonotrode 29 zugewandten Innenseite. Dieses hülsenartige, mit einer Öffnung nach Innen versehene Rückschlusselement 33 bewirkt eine Bündelung oder Verdichtung der Magnetfeldlinien oder eine Erhöhung der magnetischen Flussdichte genau in den geometrisch unterschiedlich geformten Bereichen 34, 34', 34" der Werkzeugschäfte 3B, 3'B, 3"B, wodurch die Unterscheidung der Werkzeuge 3, 3', 3" durch die Werkzeugerkennungsvorrichtung 4 begünstigt wird.

Distal anschließend an die Spule 5 ist die Beleuchtungsvorrichtung 9 angeordnet, die eine Platine 35 und eine oder mehrere optische Halbleiterelemente 10, insbesondere Leuchtdioden, umfasst. Die Platine 35 ist ringförmig geformt, so dass durch ihre zentrale Öffnung ein in die Werkzeugaufnahme 22 eingesetztes Werkzeug 3, 3', 3" ragen kann. Eine transparente Kunststoffhülse 36 deckt die Platine 35 und die Leuchtdioden 10 ab und schützt sie vor Verschmutzungen und mechanischen Belastungen.

Die elektrische Versorgung der Spule 5 und der Beleuchtungsvorrichtung 9 erfolgt über eine gemeinsame elektrische Leitung 11, die einen isolierten Metalldraht 11A und die Trägerhülse 32 durchsetzende, in die transparente Hülse 36 ragende Metallstifte 11B umfasst. Die Metallstifte 11B weisen an einem Ende Buchsen zur Aufnahme des Metalldrahts 11A oder Litzen des Metalldrahts 11A auf. Die Metallstifte 11B kontaktieren zur Versorgung der Leuchtdioden 10 die Platine 35 und die Enden der Spule 5, so dass die Spule 5 und die optischen Halbleiterelemente 10 elektrisch parallel geschaltet sind.

Bevorzugt sind zumindest die optischen Halbleiterelemente 10, die Platine 35 und die Spule 5 mit einem Vergussmaterial, zum Beispiel mit einem Kunstharz, insbesondere mit Epoxyharz, vergossen, um sie vor äußeren Einflüssen und Verschmutzungen zu schützen. Besonders bevorzugt bilden die optischen Halbleiterelemente 10, die Platine 35, die Spule 5, das Rückschlusselement 33, die transparente Hülse 36, die Trägerhülse 32 und die Stifte 11B eine vom übrigen Handstück 18 trennbare Einheit 38, die an ein Ansatzstück 37 des Handstücks 18 ansteckbar ist, wobei durch das Ansatzstück 37 auch der Draht 11A der elektrischen Leitung 11 verläuft. Zum Lösen oder Einfügen dieser Einheit 38 ist die Außenhülse 21 des Handstücks 18 zweigeteilt und umfasst einen vorderen Kappenteil 21A, der von der übrigen Außenhülse 21 trennbar ist, zum Beispiel über eine Schraubverbindung.

Anstelle des Vergießens können die optischen Halbleiterelemente 10, die Platine 35, die Spule 5, das Rückschlusselement 33 und die Stifte 11B hermetisch gekapselt sein. Dies erfolgt zumBEispiel durch Verschweißen, insbesondee durch Ultraschallschweißen, der transparenten Hülse 36 mit der Trägerhülse 32.

Die Figur 3 zeigt in Form eines schematischen Schaltbilds ein Ausführungsbeispiel einer Werkzeugerkennungsvorrichtung 4 einschließlich der Energiequelle 12. Auf der rechten Seite der Darstellung sind die Spule 5 und eine Leuchtdiode 10 abgebildet. Die Spule 5 und die Leuchtdiode 10 werden über die gemeinsame, zweiadrige elektrische Leitung 11 mit elektrischer Energie versorgt, wobei sich die Leitung 11 in zwei elektrisch parallel geschaltete Zweige aufteilt, wobei an einem Zweig die Spule 5 und am anderen Zweig die Leuchtdiode 10 angeordnet ist. Die Spule 5 ist mit den unterschiedlichen, an die Behandlungsvorrichtung 1 anschließbaren Werkzeugen 3, 3', 3" induktiv koppelbar, wodurch ein für jedes Werkzeug 3, 3', 3" spezifisches, durch die Auswerteeinheit 6 dem jeweiligen Werkzeug 3, 3', 3" zuordenbares Wechselspannungssignal generierbar ist.

Der Spule 5 ist eingangsseitig ein Kondensator 39 vorgeschaltet, so dass die Spule 5 und der Kondensator 39 gemeinsam einen Serienschwingkreis oder eine Resonanz erzeugende Vorrichtung zur Werkzeugerkennung bilden. Die Spule 5 mit dem in Serie geschalteten Kondensator 39 ist parallel zur Diode 10 geschaltet. Wird für die Spule 5 eine Schutzbeschaltung benötigt, die die Spule 5 schützt, wenn die die Leuchtdiode 10 mit Gleichspannung versorgt wird, so kann der Spule 5 ein Schutzelement, zum Beispiel ein Widerstand oder eine Diode vorgeschaltet sein.

Die Energiequelle 12 umfasst eine Gleichspannungsquelle 13, einen Sinusoszillator 14, einen Kondensator 15 für die Versorgung der Spule 5 mit Wechselspannung und einen parallel zum Kondensator 15 geschalteten Widerstand 16 für die Versorgung des zumindest einen optischen Halbleiterelements 10 mit Gleichspannung. Die Energiequelle 12 ist somit durch eine Gleichspannungsquelle 12 und eine Wechselspannungsquelle 17 gebildet, die mit der gemeinsamen elektrischen Leitung 11 verbunden sind. Über die gemeinsame elektrische Leitung 11 wird zumindest während der Werkzeugerkennung eine Mischspannung, bestehend aus überlagerter Gleichspannung und Wechselspannung, geleitet.

Die Auswerteeinheit 6 zur analogen und digitalen Signalauswertung, die bevorzugt einen Mikrocomputer aufweist, ist mit der Spule 5 verbunden und detektiert die durch die induktive Kopplung der Spule 5 mit den unterschiedlichen Werkzeugen 3, 3', 3" verursachten Änderungen der von der Wechselspannungsquelle 17 gelieferten Wechselspannungssignale. Zur Auswertung der Wechselspannungssignale und zur Werkzeugerkennung kann die Auswerteeinheit 6 an das eingangsseitige oder an das ausgangsseitige Ende der Spule 5 angeschlossen sein.

In der Figur 4 ist der Aufbau der Auswerteeinheit 6 dargestellt: Das durch die induktive Kopplung zwischen der Spule 5 und dem Werkzeug 3, 3', 3" veränderte Wechselspannungssignal U_{IN} wird zuerst einer Wandlereinheit 7, die einen Gleichrichter 7A und einen Glättungskondensator umfasst, einem Tiefpassfilter 40 und einem Spitze-Spitze-Spannungswertmesser 41 zugeführt. Das Wechselspannungssignal U_{IN} wird somit in ein Gleichspannungssignal mit zwei Spannungswerten umgewandelt, wobei die Spannungswerte in etwa gleich den Spannungswerten der Amplitudenextremwerte des Wechselspannungssignals, d.h. der maximalen und der minimalen Spannungsspitze des Wechselspannungssignals, sind. Der Spitze-Spitze-Spannungswertmesser 41 ermittelt die Differenz zwischen diesen beiden Spannungswerten, die für jedes Werkzeug 3, 3', 3" spezifisch ist, und generiert auf Basis dieser Differenz ein ebenfalls für jedes Werkzeug 3, 3', 3" spezifisches Spitze-Spitze-Messsignal Uₛₛ.

Das Messsignal Uₛₛ wird einem Messverstärker 42 und einem weiteren Tiefpassfilter 43 zugeführt, einer Pegelanpassung 44 unterzogen und durch eine den Mikroprozessor umfassende Auswerteschaltung 45 weiter verarbeitet, so dass durch eine mit der Auswerteschaltung 45 verbundene Versorgungseinheit auf das Werkzeug 3, 3', 3" abgestimmte Betriebsmittel, zum Beispiel elektrische Antriebsenergie, Kühlflüssigkeit, Druckgas, oder auf das Werkzeug 3, 3', 3" abgestimmte Betriebsmittelmengen, zum Beispiel Antriebsspannungen oder Kühlflüssigkeitsvolumenströme, abgebbar sind.

Die in den Figuren 5A und 5B dargestellten Werkzeuge oder Zahnsteinentfernungsspitzen 3', 3" weisen einen Arbeitsabschnitt 3'A, 3"A und einen Werkzeugschaft 3'B, 3"B auf. Am Werkzeugschaft 3'B, 3"B ist ein hohlzylindrischer Abschnitt 46', 46" vorgesehen, der ein Innengewinde zum Anschluss an eine Werkzeugaufnahme oder -kupplung aufweist. Der hohlzylindrische Abschnitt 46', 46" dient auch als geometrisch unterschiedlich geformter Bereich 34', 34" zur Unterscheidung der Werkzeuge 3', 3" durch die Werkzeugerkennungsvorrichtung 4. Die Bereiche 34', 34" weisen dazu unterschiedliche Durchmesser auf, da die Wandstärken der hohlzylindrischen Abschnitte 46', 46" unterschiedlich sind, wohingegen die Innenbohrungen der hohlzylindrischen Abschnitte 46', 46" mit den Innengewinden gleiche Durchmesser haben. Alternativ oder zusätzlich sind die Bereich 34', 34" unterschiedlich lang.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Medizinische, insbesondere dentale, Behandlungsvorrichtung (1), umfassend eine Antriebseinheit (2) zum Antrieb mehrerer unterschiedlicher, lösber mit der Behandlungsvorrichtung (1) verbindbarer, weichmegnetischer Werkzeuge (3, 3', 3") und eine Werkzeugerkennungsvorrichtung (4) zur Erkennung ob ein Werkzeug (3, 3', 3") an die Behandlungsvorrichtung (1) angeschlossen ist oder welches der unterschiedlichen Werkzeuge (3, 3', 3") an die Behandlungsvorrichtung (1) angeschlossen ist, wobei die Werkzeugerkennungsvorrichtung (4) eine Spule (5) und
eine Auswerteeinheit (6) umfasst und wobei die Spule (5) mit einer Wechselspannungsquelle (17) verbindbar oder verbunden ist, **dadurch gekennzeichnet, dass**
die Spule (5) mit den unterschiedlichen weichmagnetischen Werkzeugen (3, 3', 3") induktiv koppelbar ist, um zur Werkzeugerkennung die Induktivität der Spule (5) derart zu beeinflussen, dass ein für jedes Werkzeug (3, 3', 3") spezifisches, durch die Auswerteeinheit (6) dem jeweiligen Werkzeuge (3, 3', 3") zuordenbares Wechseispannungssignal generierbar ist, wobei die Auswerteeinheit (6) mit der Spule (5) verbunden ist und die durch die induktive Kopplung der Spule (5) mit den unterschiedlichen Werkzeugen (3, 3', 3") verursachten Änderungen der von der Wechseispannungsquelle (17) gelieferten Wechselspannungssignale zur Werkzeugerkennung detektiert.

2. Behandlungsvorrichtung (1) nach Anspruch 1, **dadurch gekennzeichnet, dass** die den jeweiligen Werkzeugen (3, 3', 3") zuordenbaren Wechselspannungssignale unterschiedliche Amplituden mit unterschiedlichen Amplitudenextremwerten aufweisen und die Auswerteeinheit (6) zur Unterscheidung der Werkzeuge (3, 3', 3") auf der Basis zumindest eines Amplitudenextremwerts ausgebildet ist.

3. Behandlungsvorrichtung (1) nach Anspruch 1 oder 2, **dadurch gekennzeichnet, dass** die Auswertereinheit (6) eine Wandlereinheit (7) aufweist, die insbesondere zumindest einen Gleichrichter (7A) und einen Glättungskondensator umfasst, welche die den jeweiligen Werkzeugen (3, 3', 3") zuordenbaren Wechselspannungssignale in Gleichspannungssignale wandelt.

4. Behandlungsvorrichtung (1) nach einem der vorherstehenden Ansprüche, **dadurch gekennzeichnet, dass**
die Behandlungsvorrichtung (1) einen Satz unterschiedlicher Werkzeuge (3, 3', 3") umfasst, deren Werkzeugschäfte (3B, 3'B, 3"B) eine unterschiedliche Geometrie und
/ oder unterschledliche Materialien aufweisen.

5. Behandlungsvorrichtung (1) nach einem der vorherstehenden Ansprüche, **dadurch gekennzeichnet, dass**
die Antriebseinheit (2) einen, insbesondere piezoelektrischen, Schwingungserreger (8) umfasst, der In Abhängigkeit des mit der Behandlungsvorrichtung (1) verbundenen und von der Werkzeugerkennungsvorrichtung (4) erkannten Werkzeugs (3, 3', 3") mit auf das Werkzeug (3, 3', 3") abgestimmter Antriebsenergie, Antriebsleistung oder Antriebsspannung versorgbar Ist.

6. Behandlungsvorrichtung (1) nach einem der vorherstehenden Ansprüche, **dadurch gekennzeichnet, dass**
die Behandlungsvorrichtung (1) eine Beleuchtungsvorrichtung (9) mit zumindest einem optischen Halbleiterelement (10) aufweist, wobei zur Versorgung der Beleuchtungsvorrichtung (9) und der Spule (5) der Werkzeugerkennungsvorrichtung (4) mit elektrischer Energie eine gemeinsame elektrische Leitung (11, 11 A, 11B) vorgesehen ist und wobei die Spule (6) und die Beleuchtungsvorrichtung (9) parallel zueinander geschaltet sind.

7. Behandlungsvorrichtung (1) nach Anspruch 6, **dadurch gekennzeichnet, dass** die gemeinsame elektrische Leitung (11, 11A, 11 B) mit einer Energiequelle (12) verbindbar oder verbunden ist, die zur Versorgung der Beleuchtungsvorrichtung (9) Gleichspannung und zur Versorgung der Spule (5) Wechselspannung bereitstellt.

8. Behandlungsvorrichtung (1) nach Anspruch 7, **dadurch gekennzeichnet, dass** die Energiequelle (12) eine Gleichspannungsquelle (13), einen Sinusoszillator (14), einen Kondensator (15) für die Versorgung der Spule (5) mit Wechselspannung und einen parallel zum Kondensator (15) geschalteten Widerstand (16) für die Versorgung des zumindest einen optischen Halbleiterelements (10) mit Gleichspannung aufweist.

9. Behandlungsvorrichtung (1) nach Anspruch 7 oder 8, **gekennzeichnet durch** ein Schaltelement zum Schalten der Energieversorgung für die Spule (5) und die Beleuchtungsvorrichtung (9) zwischen einem ersten Betriebszustand, bei dem die Werkzeugerkennung erfolgt, und einem zweiten Betriebszustand, wobei während des ersten Betriebszustands die Gleichspannung einen niedrigen Spannungswert und die Wechselspannung einen hohen Spannungswert aufweisen und wobei während des zweiten Betriebszustands die Gleichspannung einen hohen Spannungswert und die Wechselspannung einen niedrigen Spannungswert aufwelsen.

10. Behandlungsvorrichtung (1) nach Anspruch 9, **dadurch gekennzeichnet, dass** der niedrige Spannungswert der Gleichspannung pro optischem Halbleiterelement (10) zwischen etwa 0,9 V - 1,5 V, bevorzugt etwa 1,1 - 1,2 V, beträgt, dass der hohe Spannungswert der Gleichspannung pro optischem Halbleiterelement (10) zwischen etwa 2,5 V - 3,3 V, bevorzugt etwa 3,0 - 3,2 V, beträgt, dass der niedrige Spannungswert der Wechselspannung zwischen etwa 0,0 V - 0,5 V beträgt und dass der hohe Spannungswert der Wechselspannung zwischen etwa 10 V - 18 V, bevorzugt etwa 15 V, beträgt.

11. Behandlungsvorrichtung (1) nach einem der vorherstehenden Ansprüche, **dadurch gekennzeichnet, dass**
die Auswerteeinheit (6) einen Speicher, in dem für jedes Werkzeug (3, 3', 3") ein charakteristischer Wert hinterlegt ist, insbesondere ein charakteristischer Spannungswert, und einen Komparator aufweist, der zur Erkennung des mit der Behandlungsvorrichtung (1) verbundenen Werkzeugs den hinterlegten Wert mit dem für jedes Werkzeug (3, 3', 3") spezifischen Wechselspannungssignal oder einem davon abgeleiteten Signal vergleicht.

12. Behandlungsvorrichtung (1) nach Anspruch 11, **dadurch gekennzeichnet, dass**
die Auswerteeinheit (6) zur Abgabe eines spezifischen Steuersignals für jedes Werkzeug (3, 3', 3") an eine Versorgungseinheit ausgebildet ist, so dass durch die Versorgungseinheit auf das Werkzeug (3, 3', 3") abgestimmte Betriebsmittel, zum Beispiel elektrische Antriebsenergie, Kühlflüssigkeit, Druckgas, oder auf das Werkzeug (3, 3', 3") abgestimmte Betriebsmittelmengen abgebbar sind.

13. Behandlungsvorrichtung (1) nach einem der vorherstehenden Ansprüche, **dadurch gekennzeichnet, dass**
die Spule (5) mit ihrem eingangsseitigen Ende mit einer Wechselspannungsquelle und mit ihrem ausgangsseitigen Ende mit einer Auswerteeinheit (6) zur Auswertung des ausgangsseitig der Spule (5) anliegenden Wechselspannungssignals verbindbar oder verbunden ist.

14. Verfahren zur Erkennung, ob ein Werkzeug (3, 3', 3") an eine medizinische, insbesondere dentale, Behandlungsvorrichtung (1) nach einem der vorherstehenden Ansprüche angeschlossen ist, oder welches der unterschiedlichen Werkzeuge (3, 3', 3") an die Behandlungsvorrichtung (1) angeschlossen ist, **dadurch gekennzeichnet, dass**
die Spule (5) mit einem der unterschiedlichen weichmagnetischen Werkzeuge (3, 3', 3") induktiv gekoppelt wird, um zur Werkzeugerkennung die Induktivität der Spule (5) derart zu beeinflussen, dass ein für jedes Werkzeug (3, 3', 3") spezifisches, durch die Auswerteeinheit (6) dem jeweiligen Werkzeug (3, 3', 3") zuordenbares Wechselspannungssignal generiert wird, wobei die Auswerteeinheit (6) mit der Spule (5) verbunden ist und die durch die induktive Kopplung der Spule (5) mit den unterschiedlichen Werkzeugen (3, 3', 3") verursachten Änderungen der von der Wechselspannungsquelle (17) gelieferten Wechselspannungssignale zur Werkzeugerkennung detektiert.

15. Verfahren nach Anspruch 14, **dadurch gekennzeichnet, dass**
die Behandlungsvorrichtung (1) eine Beleuchtungsvorrichtung (9) mit zumindest einem optischen Halbleiterelement (10) aufweist, wobei die Beleuchtungsvorrichtung (9) und die Spule (5) der Werkzeugerkennungsvorrichtung (4), die parallel zueinander angeordnet sind, über eine gemeinsame elektrische Leitung (11, 11A, 11B) mit elektrischer Energie versorgt werden.

## Claims

1. A medical, in particular dental, treatment device (1), comprising a drive unit (2) for driving multiple different soft-magnetic tools (3, 3', 3") detachably connectable to the treatment device (1) and a tool recognition device (4) for recognizing whether a tool (3, 3', 3") is connected to the treatment device (1) or which of the different tools (3, 3', 3") is connected to the treatment device (1), wherein the tool recognition device (4) comprises a coil (5) und an evaluation unit (6) and wherein the coil (5) is connectable or connected to an AC voltage source (17), **characterized in that** the coil (5) is inductively couplable to the different soft-magnetic tools (3, 3', 3") to affect the inductance of the coil (5) for the tool recognition such that an AC voltage signal, which is specific for each tool (3, 3', 3") and can be assigned to the respective tool (3, 3', 3") by the evaluation unit (6) can be generated, wherein the evaluation unit (6) is connected to the coil (5) and detects the changes in the AC voltage signals supplied by the AC voltage source (17) due to the inductive coupling of the coil (5) to the different tools (3, 3', 3") for the tool recognition.

2. The treatment device (1) according to claim 1, **characterized in that** the AC voltage signals, which can be assigned to the respective tools (3, 3', 3"), comprise different amplitudes with different amplitude extreme values, and the evaluation unit (6) is designed for differentiating the tools (3, 3', 3") on the basis of at least one amplitude extreme value.

3. The treatment device (1) according to claim 1 or 2, **characterized in that** the evaluation unit (6) comprises a converter unit (7), which has in particular at least one rectifier (7A) and one smoothing capacitor, for converting the AC voltage signals that can be assigned to the respective tools (3, 3', 3") into DC voltage signals.

4. The treatment device (1) according to one of the preceding claims, **characterized in that**
the treatment device (1) comprises a set of different tools (3, 3', 3") having tool shafts (3B, 3'B, 3"B) of different geometries and/or different materials.

5. The treatment device (1) according to one of the preceding claims, **characterized in that**
the drive unit (2) includes a vibration generator (8), in particular a piezoelectric vibration generator, which can be supplied with driving energy, driving power or driving voltage tailored to the tool (3, 3', 3") as a function of the tool (3, 3', 3") that is connected to the treatment device (1) and is recognized by the tool recognition device (4).

6. The treatment device (1) according to one of the preceding claims, **characterized in that**
the treatment device (1) comprises an illumination device (9) having at least one optical semiconductor element (10), wherein a shared electric line (11, 11A, 11B) is provided for supplying electric energy to the illumination device (9) and to the coil (5) of the tool recognition device (4), and wherein the coil (5) and the illumination device (9) are connected in parallel to one another.

7. The treatment device (1) according to claim 6, **characterized in that**
the shared electric line (11, 11A, 11 B) is connected or connectable to a power source (12), which supplies DC voltage for the power supply to the illumination device (9) and supplies AC voltage for the power supply to the coil (5).

8. The treatment device (1) according to claim 7, **characterized in that**
the power source (12) comprises a DC voltage source (13), a sine-wave oscillator (14), a capacitor (15) for supplying AC voltage power to the coil (5), and a resistor (16) that is connected in parallel to the capacitor (15) for supplying DC voltage power to the at least one optical semiconductor element (10).

9. The treatment device (1) according to claim 7 or 8, **characterized by**
a switch element for switching the power supply for the coil (5) and the illumination device (9) between a first operating state, in which the tool recognition is performed, and a second operating state, wherein the DC voltage has a low voltage value and the AC voltage has a high voltage value during the first operating state, and wherein the DC voltage has a high voltage value and the AC voltage a low voltage value during the second operating state.

10. The treatment device (1) according to claim 9, **characterized in that**
the low voltage value of the DC voltage per optical semiconductor element (10) is between approximately 0.9 V - 1.5 V, preferably approximately 1.1 V -1.2 V, the high voltage value of the DC voltage per optical semiconductor element (10) is between approximately 2.5 V - 3.3 V, preferably approximately 3.0 V - 3.2 V, the low voltage value of the AC voltage is approximately 0.0 V - 0.5 V, and the high voltage value of the AC voltage is approximately 10 V - 18 V, preferably approximately 15 V.

11. The treatment device (1) according to one of the preceding claims, **characterized in that**
the evaluation unit (6) comprises a memory in which a characteristic value, in particular a characteristic voltage value, is stored for each tool (3, 3', 3"), and a comparator which compares the stored value with the AC voltage signal specific for each tool (3, 3', 3") or a signal derived therefrom for the recognition of the tool connected to the treatment device (1).

12. The treatment device (1) according to claim 11, **characterized in that**
the evaluation unit (6) is designed for delivering a specific control signal for each tool (3, 3', 3") to a supply unit, so that resources tailored to the tool (3, 3', 3"), for example, electric driving power, cooling liquid, compressed gas, or quantities of operating resources tailored to the tool (3, 3', 3") can be dispensed by the supply unit.

13. The treatment device (1) according to one of the preceding claims, **characterized in that**
the coil (5) is connected or connectable to an AC voltage source via its input end and to an evaluation unit (6) via its output end for evaluating the AC voltage signal at the output end of the coil (5).

14. A method for recognizing whether a tool (3, 3', 3") is connected to a medical, in particular dental, treatment device (1) according to any one of the preceding claims, or for recognizing which of the different tools (3, 3', 3") is connected to the treatment device (1), **characterized in that**
the coil (5) is inductively coupled to one of the different soft-magnetic tools (3, 3', 3") to affect the inductance of the coil (5) for the tool recognition such that an AC voltage signal, which is specific for each tool (3, 3', 3") and can be assigned to the respective tool (3, 3', 3") by the evaluation unit (6) is generated, wherein the evaluation unit (6) is connected to the coil (5) and detects the changes in the AC voltage signals supplied by the AC voltage source (17) due to the inductive coupling of the coil (5) to the different tools (3, 3', 3") for the tool recognition.

15. The method according to claim 14, **characterized in that**
the treatment device (1) has an illumination device (9) with at least one optical semiconductor element, wherein the illumination device (9) and the coil (5) of the tool recognition device (4), which are arranged in parallel with one another, are supplied with electric energy via a shared electric line (11, 11A, 11 B).

## Revendications

1. Dispositif de traitement médical (1), en particulier dentaire, comprenant une unité motrice (2) permettant d'entraîner (1) plusieurs outils différents magnétiquement souples (3, 3', 3") pouvant être reliés de manière amovible au dispositif de traitement (4) et un dispositif de détection d'outil (4) permettant de détecter si un outil (3, 3', 3") est raccordé au dispositif de traitement (1) ou lequel des différents outils (3, 3', 3") est raccordé au dispositif de traitement (1), le dispositif de détection d'outil (4) comprenant une bobine (5) et une unité d'exploitation (6) et la bobine (5) pouvant être ou étant raccordée à une source de tension alternative (17), **caractérisé en ce que** la bobine (5) peut être couplée par induction aux différents outils magnétiquement souples (3, 3', 3") afin d'influer sur l'inductance de la bobine (5) pour détecter l'outil de manière à ce que puisse être généré un signal de tension alternative spécifique à chaque outil (3, 3', 3") et associable par l'unité d'exploitation (6) à l'outil respectif (3, 3', 3"), l'unité d'exploitation (6) étant raccordée à la bobine (5) et détecte les modifications dues au couplage inductif de la bobine (5) avec les différents outils (3, 3', 3") des signaux de tension alternative délivrés par la source de tension alternative (17) pour la détection de l'outil.

2. Dispositif de traitement (1) selon la revendication 1, **caractérisé en ce que**
les signaux de tension associables aux outils respectifs (3, 3', 3") présentent des amplitudes différentes avec diverses valeurs extrêmes d'amplitude et que l'unité d'exploitation (6) est conçue pour différencier les outils (3, 3', 3") en partant d'au moins une valeur extrêmes d'amplitude.

3. Dispositif de traitement (1) selon la revendication 1 ou 2, **caractérisé en ce que**
l'unité d'exploitation (6) présente une unité de conversion (7) qui comprend en particulier au moins un redresseur (7A) et un condensateur de lissage qui convertit les signaux de tension alternative associables aux outils respectifs (3, 3', 3") en signaux de tension continue.

4. Dispositif de traitement (1) selon une des revendications précédentes, **caractérisé en ce que**
le dispositif de traitement (1) comprend un ensemble d'outils différents (3, 3', 3") dont les arbres d'outil (3B, 3'B, 3"B) présentent une géométrie différente et/ou différents matériaux.

5. Dispositif de traitement (1) selon une des revendications précédentes, **caractérisé en ce que**
l'unité motrice (2) présente un excitateur vibratoire (8), en particulier piézoélectrique qui, en fonction de l'outil (3, 3', 3") raccordé au dispositif de traitement (1) et détecté
par le dispositif de détection d'outil (4), peut être alimenté en énergie motrice, puissance motrice ou tension motrice adaptée à l'outil (3, 3', 3").

6. Dispositif de traitement (1) selon une des revendications précédentes, **caractérisé en ce que**
le dispositif de traitement (1) présente un dispositif d'éclairage (9) doté d'au moins un élément semi-conducteur optique (10), un câble électrique commun (11, 11A, 11B) étant prévu pour alimenter le dispositif d'éclairage (9) et la bobine (5) du dispositif de détection d'outil (4) en énergie électrique et la bobine (5) et le dispositif d'éclairage (9) étant branchés parallèlement.

7. Dispositif de traitement (1) selon la revendication 6, **caractérisé en ce que**
le câble électrique commun (11, 11A, 11B) peut être ou est raccordé à une source d'énergie (12) qui fournit de la tension continue pour alimenter le dispositif d'éclairage (9) et de la tension alternative pour alimenter la bobine (5).

8. Dispositif de traitement (1) selon la revendication 7, **caractérisé en ce que**
la source d'énergie (12) comprend une source de tension continue (13), un oscillateur sinusoïdal (14), un condensateur (15) pour alimenter la bobine (5) en tension alternative et une résistance (16) branchée parallèlement au condensateur (15) pour alimenter l'au moins un élément semi-conducteur optique (10) en tension continue.

9. Dispositif de traitement (1) selon la revendication 7 ou 8, **caractérisé par**
un élément de commutation pour commuter l'alimentation en énergie pour la bobine (5) et le dispositif d'éclairage (9) entre un premier état de fonctionnement dans lequel la détection d'outil a lieu et un second état de fonctionnement, dans lequel, pendant le premier état de fonctionnement, la tension continue présente une valeur de tension faible et la tension alternative une valeur de tension élevée et, pendant le second état de fonctionnement, la tension continue présente une valeur de tension élevée et la tension alternative une valeur de tension faible.

10. Dispositif de traitement (1) selon la revendication 9, **caractérisé en ce que**
la valeur de tension faible de la tension continue par élément semi-conducteur optique (10) est comprise entre environ 0,9 V et 1,5 V, de préférence environ 1,1 et 1,2 V, que la valeur de tension élevée de la tension continue (10) par élément semi-conducteur optique (10) est comprise entre environ 2,5 V et 3,3 V, de préférence environ 3,0 et 3,2 V, que la valeur de tension faible de la tension alternative est comprise entre environ 0,0 V et 0,5 V et que la valeur de tension élevée de la tension alternative est comprise entre environ 10 V et 18 V et s'élève de préférence à environ 15 V.

11. Dispositif de traitement (1) selon une des revendications précédentes, **caractérisé en ce que**
l'unité d'exploitation (6) présente une mémoire dans laquelle est enregistrée pour chaque outil (3, 3', 3") une valeur caractéristique, en particulier une valeur de tension caractéristique et un comparateur qui, pour détecter l'outil raccordé au dispositif de traitement (1), compare la valeur enregistrée au signal de tension alternative spécifique à chaque outil (3, 3', 3") ou à un signal en dérivant.

12. Dispositif de traitement (1) selon la revendication 11, **caractérisé en ce que**
l'unité d'exploitation (6) est conçue pour émettre un signal de commande spécifique pour chaque outil (3, 3', 3") vers une unité d'alimentation de manière à ce que des consommables adaptés à l'outil (3, 3', 3"), par exemple de l'énergie motrice, du liquide de refroidissement, du gaz comprimé ou des quantités de consommables adaptées à l'outil (3, 3', 3") puissent être délivrés par l'unité d'alimentation.

13. Dispositif de traitement (1) selon une des revendications précédentes, **caractérisé en ce que**
la bobine (5) peut être ou est raccordée par son extrémité située côté entrée à une source de tension alternative et par son extrémité située côté sortie à une unité d'exploitation (6) permettant d'exploiter le signal de tension alternative appliqué du côté sortie de la bobine (5).

14. Procédé permettant de détecter si un outil (3, 3', 3") est raccordé à un dispositif de traitement médical (1), en particulier dentaire, selon une des revendications précédentes ou lequel des outils différents (3, 3', 3") est raccordé au dispositif de traitement (1), **caractérisé en ce que**
la bobine (5) est couplée par induction à un des différents outils magnétiquement souples (3, 3', 3") afin d'influer sur l'inductance de la bobine (5) pour détecter l'outil de manière à ce que puisse être généré un signal de tension alternative spécifique à chaque outil (3, 3', 3") et associable par l'unité d'exploitation (6) à l'outil respectif (3, 3', 3"), l'unité d'exploitation (6) étant raccordée à la bobine (5) et détecte les modifications dues au couplage inductif de la bobine (5) avec les différents outils (3, 3', 3") des signaux de tension alternative délivrés par la source de tension alternative (17) pour la détection de l'outil.

15. Procédé selon la revendication 14, **caractérisé en ce que**
le dispositif de traitement (1) présente un dispositif d'éclairage (9) doté d'au moins un élément semi-conducteur optique (10), le dispositif d'éclairage (9) et la bobine (5) du dispositif de détection d'outil (4), qui sont disposés parallèlement, étant alimentés en énergie électrique par un câble électrique commun (11, 11A, 11 B).
